# FASCICULE DE BREVET EUROPEEN

(11) **EP 4 132 415 B1**
(45) Date de publication et mention de la délivrance du brevet: **21.01.2026**
(21) Numéro de dépôt: 21718092.6
(22) Date de dépôt: 08.04.2021
(51) Int. Cl.: A61C 8/00, A61B 17/84, A61B 17/68, A61B 17/70, A61B 17/86

(54) **IMPLANT D'ANCRAGE OSSEUX À EXPANSION OPTIMISÉE**
KNOCHENVERANKERUNGSIMPLANTAT MIT OPTIMIERTER EXPANSION
BONE ANCHORING IMPLANT WITH OPTIMISED EXPANSION

(30) Priorité: 09.04.2020 FR 2003577
(43) Date de publication de la demande: 15.02.2023
(73) Titulaire: Lock-In SA, 1180 Rolle (CH)
(72) Inventeur: LACAZE, Guillaume, 1278 La Rippe (CH)
(74) Mandataire: Gsmart
(86) Numéro de dépôt international: PCT/EP2021/059209
(87) Numéro de publication internationale: WO 2021/204959

(56) Documents cités:
- CH-A2- 715 121
- US-A- 5 611 688
- US-A1- 2012 265 258
- US-A1- 2017 119 503

## Description

### DOMAINE TECHNIQUE ET OBJET DE L'INVENTION

La présente invention se rapporte au domaine des implants osseux destinés à des applications dentaires, orthopédiques, chirurgicales ou ostéoplastiques, tels que des vis orthopédiques seules ou avec des plaques, des implants dentaires ou ligamentaires pour des articulations tels que par exemple les hanches, les coudes, les chevilles, les épaules et les genoux, ou spinaux rachidiens par exemple pour les vertèbres. Ces domaines d'application sont donnés à titre d'exemple et ne sont pas restrictifs quant à la portée de la présente invention.

Plus spécifiquement l'invention concerne un implant osseux dont l'implantation dans le tissu osseux est stabilisé par une expansion notamment dans la partie spongieuse de l'os.

### ETAT DE LA TECHNIQUE

Un implant d'ancrage osseux est généralement constitué d'un corps de forme allongée destiné à être implanté dans un logement formé dans un tissu osseux, tel que l'os de la mâchoire pour une application dentaire ou dans une vertèbre par exemple.

Il est important que l'implant d'ancrage osseux puisse être introduit facilement dans le tissu osseux, sans créer de dommages, et que le dispositif d'ancrage à l'intérieur du tissu osseux soit stable. En effet, les dispositifs d'implant osseux actuels ne permettent pas un ancrage fiable sans engendrer de fissures ou de dommages plus importants que ne le nécessite la taille du dispositif lui-même dans le tissu osseux. Il est nécessaire que la fixation dans l'implant d'ancrage osseux soit fiable et stable, car de nombreuses techniques thérapeutiques reposent aujourd'hui sur la croissance osseuse qui nécessite généralement que les dispositifs ancrés dans le tissu osseux restent le plus immobile possible.

En outre, il est aussi nécessaire que l'implantation dans le tissu osseux soit facile à réaliser pour éviter tout risque de mauvais positionnement de l'implant d'ancrage osseux, qui pourrait notamment être dû à une difficulté de positionnement ou d'implantation dans l'os.

L'état de la technique comprend le document de brevet EP2603163 B1, qui décrit un implant endo-osseux à ancrage amélioré apte à être implanté dans un tissu osseux et comportant un dispositif de fixation comprenant une partie dite d'accrochage dans le tissu osseux, et une partie dite d'expansion, ces deux parties étant mobiles l'une par rapport à l'autre. L'invention mentionnée dans ce brevet comprend également des moyens de liaison mécanique coopérants disposés, d'une part, sur la partie d'accrochage et, d'autre part, sur la partie d'expansion, de sorte que la mobilité relative des deux parties comprend au moins un degré de liberté et qu'un déplacement relatif desdites deux parties provoque un élargissement de la partie d'accrochage, ledit élargissement provoquant l'ancrage de la partie d'accrochage dans le tissu osseux. L'implant osseux décrit dans ce brevet trouve particulièrement application dans le domaine dentaire.

Cependant, une telle solution présente des inconvénients car l'implant osseux proposé présente une portion conique uniquement sur sa portion distale qui s'ouvre sur des fentes débouchantes. L'expansion de l'implant osseux dans le tissu osseux se fait alors de façon radiale et homothétique ce qui le rend moins stable notamment lors de son retrait du tissu osseux qui provoque d'abord un recul de l'implant osseux avant de pouvoir être retirer.

L'invention vise donc à résoudre ces inconvénients en proposant un implant osseux apte à être implanté et immobilisé dans le tissu osseux de façon stable.

Le document US 5 611 688 A divulgue un implant d'ancrage osseux à expansion comprenant un corps tubulaire fileté intérieurement et extérieurement et une tige de profil extérieur complémentaire audit profil intérieur dudit corps tubulaire, la pénétration de la tige dans le corps tubulaire engendrant l'expansion radiale dudit corps tubulaire.

### PRESENTATION GENERALE DE L'INVENTION

La présente invention a donc pour objet de pallier les inconvénients de l'art antérieur en proposant un implant d'ancrage osseux, ci-après dénommé implant osseux, facilement implantable dans le tissu osseux, stable, et qui se retire également facilement du tissu osseux.

Pour parvenir à ce résultat, la présente invention concerne un implant d'ancrage osseux à expansion optimisée, comprenant :
Un corps tubulaire s'étendant entre une portion proximale possédant un premier diamètre intérieur, et une portion distale possédant un second diamètre intérieur inférieur audit premier diamètre intérieur, ces deux portions définissant un axe longitudinal (L) et lesdits premier et second diamètres intérieur définissant un profil intérieur dudit corps tubulaire, et comprenant, d'une part, au moins un premier filetage à l'intérieur du corps tubulaire et, d'autre part, au moins un second filetage à l'extérieur du corps tubulaire,

Une tige s'étendant entre une portion proximale et une portion distale sur un axe colinéaire à l'axe (L) et présentant, d'une part, le long dudit axe longitudinal (L), un profil extérieur complémentaire audit profil intérieur dudit corps tubulaire et, d'autre part, au moins un filetage extérieur dont le pas de vis est inversé par rapport audit second filetage extérieur du corps tubulaire,

L'implant étant expansible entre, d'une part, une configuration de repos dans laquelle un mécanisme de butée verrouille réciproquement ledit corps tubulaire et ladite tige grâce à l'inversion de ces deux pas de vis, et, d'autre part, une configuration expandue obtenue par l'actionnement desdits filetages complémentaires intérieur et extérieur du corps tubulaire et de la tige réciproquement, provoquant la pénétration de la tige dans le corps tubulaire et engendrant l'expansion dudit corps tubulaire, grâce au diamètre extérieur de la tige qui est supérieur au diamètre intérieur du corps tubulaire, au moins sur une portion distale, par déformation du corps tubulaire lors de la pénétration de la tige dans le corps tubulaire,

Les profils extérieur de la tige et intérieur du corps tubulaire étant complémentaires, de sorte qu'ils fournissent, en configuration expandue :
Un point d'appui proximal supporté par la complémentarité du diamètre externe de la tige avec le diamètre interne du corps tubulaire,
Un point d'appui distal supporté pas la coopération entre le corps tubulaire dont le diamètre interne se rétrécit vers la portion distale jusqu'à être inférieur au diamètre externe de la tige,
Un point d'appui « central » localisé entre ces deux points d'appuis (au niveau proximal et distal), formé par la coopération entre le diamètre externe de la tige et le diamètre interne du corps tubulaire qui induisent, en configuration expandue, un diamètre externe du corps tubulaire au niveau « central » qui est supérieur au diamètre externe du corps tubulaire au niveau du point d'appui proximal.

Selon une particularité, ledit diamètre externe du corps tubulaire au niveau « central » est supérieur au diamètre externe du corps tubulaire au niveau du point d'appui distal. Selon une particularité, le point d'appui distal est formé sur au moins une portion de la portion distale complémentaire du profil extérieur de la tige.

Selon une autre particularité, le point d'appui distal est formé par ladite portion distale comprenant une pointe dont le profil extérieur est complémentaire du profil intérieur de la portion distale du corps tubulaire.

Selon une autre particularité, le point d'appui central est formé par au moins une nervure faisant saillie à l'intérieur du corps tubulaire, sur une portion intermédiaire entre la portion distale et la portion proximale, de sorte que ladite nervure coopère, lors de l'expansion, avec la surface extérieure de la tige entre sa portion proximale et sa portion distale.

Selon une autre particularité, le mécanisme de butée réciproque comporte une nervure ou un épaulement ou une saillie à l'intérieur du corps tubulaire complémentaire, du diamètre extérieur de la tige, ou une nervure sur l'intérieur du corps tubulaire, complémentaire d'une rainure ou d'un épaulement sur la tige.

Selon une particularité, le mécanisme de butée réciproque comporte une nervure faisant saillie à l'intérieur du corps tubulaire complémentaire d'un épaulement ou d'une entaille situé sur la pointe de la tige.

Selon une autre particularité, le point d'appui central et le mécanisme de butée verrouillant réciproquement le corps tubulaire et la tige sont formés par les mêmes éléments coopérant, respectivement, en configuration expandue ou en configuration de repos.

Selon une autre particularité, le diamètre extérieur de la tige est supérieur au diamètre intérieur du corps tubulaire, par au moins un rétrécissement sur une portion distale.

Selon une autre particularité, ledit au moins un rétrécissement est situé, par rapport à la portion proximale et selon l'axe longitudinal (L), à une distance déterminée en fonction de la profondeur, dans le tissu osseux, à laquelle ladite expansion est souhaitée.

Selon une autre particularité, le corps tubulaire comprend sur sa portion distale une portion tronconique dont le diamètre intérieur est inférieur au diamètre extérieur de la tige.

Selon une autre particularité, la portion tronconique présente un filetage avec une âme conique permettant au corps tubulaire de s'enfoncer profondément dans l'os.

Selon une autre particularité, la portion distale comporte des encoches auto-taraudantes.

Selon une autre particularité, la portion distale comporte des fentes longitudinales débouchantes permettant l'expansion du corps tubulaire.

Selon une autre particularité, il y a autant d'encoches auto-taraudantes que de fentes longitudinales débouchantes.

Selon une autre particularité, la portion distale comporte des fentes longitudinales non débouchantes permettant l'expansion du corps tubulaire.

Selon une autre particularité, la tige comprend au moins un marqueur de distance pour visualiser le moment où le vissage de la vis dans le corps tubulaire doit être réalisé dans le sens opposé au vissage du corps tubulaire dans le tissu osseux.

### PRESENTATION DES FIGURES

D'autres caractéristiques et avantages de l'invention apparaîtront à la lecture de la description détaillée des modes de réalisation de l'invention, donnés à titre d'exemple uniquement, et en référence aux dessins qui montrent :
[Fig. 1a], [Fig. 1b] et [Fig. 2] représentent une vue détaillée des éléments qui composent l'implant osseux selon l'invention.
[Fig. 3a] représente une vue détaillée du corps tubulaire avant l'expansion selon l'invention.
[Fig. 3b] et [Fig. 3c] représentent une vue détaillée du corps tubulaire après l'expansion selon l'invention.
[Fig. 4a] et [Fig. 4b] représentent un schéma d'une coupe de l'intérieur de la tige pénétrant dans le corps tubulaire au niveau de la butée réciproque, selon l'invention.
[Fig. 5a] et [Fig. 5b] représentent un schéma d'une vue de l'intérieur de l'implant osseux en position expandue selon l'invention.
[Fig. 6] et [Fig. 7] représentent une vue de l'extérieur de l'implant osseux en position expandue selon l'invention.
[Fig. 8] et [Fig. 9] représentent une vue du corps tubulaire expandu selon l'invention.
[Fig. 10] représente un schéma d'une coupe de l'intérieur du corps tubulaire comprenant la tige, en position expandue, selon l'invention.

### DESCRIPTION DETAILLE D'UNE FORME DE REALISATION DE L'INVENTION

Divers modes de réalisation de l'invention sont décrits ci-dessous notamment en référence aux figures illustratives et non limitatives.

La présente demande concerne l'implantation d'un implant osseux dans un tissu osseux.

On notera ici que l'on désigne par le terme « implantation » le fait d'introduire l'implant osseux dans le tissu osseux, généralement par vissage. L'implantation proposée dans la présente demande désigne une introduction suffisamment solide et stable de l'implant osseux pour assurer un bon maintien de cet implant osseux dans le tissu osseux.

Par ailleurs, le terme « tissu(s) osseux » désigne généralement tous types d'os, qu'il s'agisse d'os compact (comme par exemple l'os cortical ou le périoste) ou d'os spongieux (mou, poreux), car le système d'implant osseux de la présente demande est implantable dans tout type de tissu osseux.

En outre, les termes utilisés ne doivent pas être interprétés dans leur acception générale mais plutôt à la lumière des considérations fonctionnelles détaillées dans la présente demande.

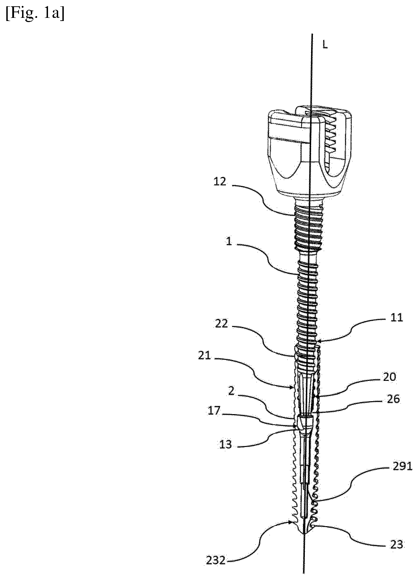

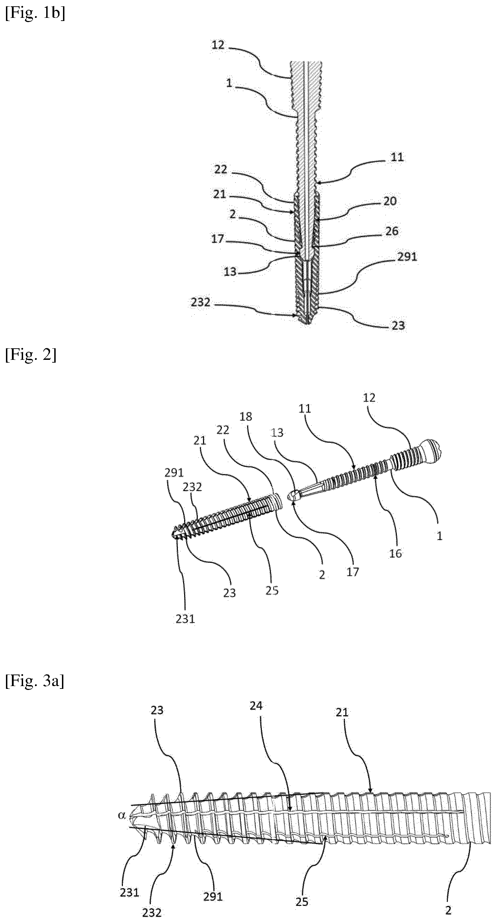

[Fig. 1a], [Fig. 1b] et [Fig. 2] sont des exemples de réalisation illustratifs et non limitatifs de l'implant osseux.

Comme par exemple représenté sur [Fig. 1a], [Fig. 1b] et [Fig. 2], un implant osseux comprend : un corps tubulaire (2) s'étendant selon un axe longitudinal (L) entre une portion proximale (22) et une portion distale (23) qui comprend, d'une part, au moins un premier filetage (20) à l'intérieur du corps tubulaire (2) et, d'autre part, au moins un second filetage (21) à l'extérieur du corps tubulaire (2).

Dans la présente demande, le terme le corps tubulaire (2) désigne généralement un cylindre généralisé (generalized cylinder en anglais) creux

Dans certains modes de réalisation, l'implant osseux comprend aussi une tige (1) s'étendant également selon ledit axe longitudinal (L) entre une portion proximale (12) et une portion distale (13) et présentant, d'une part, le long dudit axe longitudinal (L), un profil extérieur complémentaire au profil intérieur dudit corps tubulaire (2) et, d'autre part, au moins un filetage (11) extérieur dont le pas de vis est inversé par rapport audit second filetage extérieur (21) du corps tubulaire (2).

Les termes « proximal » et « distal » désignent dans la présente demande, respectivement, la partie où le dispositif d'implantation est tenu pour permettre son implantation dans le tissu osseux, et la partie qui est implantée en premier dans le tissu osseux (à l'opposé de la portion proximale).

Les termes « portions » proximale et distale désignent dans la présente demande les parties situées à proximité des extrémités distales et proximales.

Dans certains modes de réalisation, la portion proximale (12) de la tige (1) est directement implantée dans l'os cortical.

Dans certains modes de réalisation, l'extrémité proximale de la tige (1) comprend un moyen d'actionnement permettant de visser la tige (1), ledit moyen d'actionnement comprenant une structure de toute forme souhaitable par le praticien selon l'utilisation qui en sera faite, comme par exemple représenté sur [Fig. 1a]. Le moyen d'actionnement étant par exemple un trou à six pans ou un torx ou un cruciforme ou tout autre moyen d'actionnement, et l'extrémité proximale de la tige (1) pourra avoir diverses formes en fonction de la destination souhaitable pour l'implant d'ancrage osseux (tête de fixation de barre d'ostéosynthèse polyaxiale ou non, ou fixation de plaque ou tout autre dispositif).

Dans certains modes de réalisation, la tige (1) comprend une canule traversant la tige (1) pour permettre au praticien d'injecter par exemple du ciment, s'il estime cela nécessaire.

Dans certains modes de réalisation, le second filetage (21) à l'extérieur du corps tubulaire (2) permet l'ancrage osseux. Le terme « ancrage osseux » utilisé dans la présente demande désigne de façon générale divers types de dispositifs comprenant au moins un élément destiné à pénétrer le tissu osseux selon un trajet rectiligne, sous l'action d'une poussée généralement exercée sous la forme de frappes, d'impacts, ou de vissages répétés. Il est connu qu'un filetage d'ancrage osseux possède une hauteur de filet généralement supérieure à celle d'un filetage mécanique pour assurer un meilleur ancrage. En outre, un filetage d'ancrage osseux est généralement différent d'un filetage mécanique et l'Homme du métier sait, qu'en fonction du type d'os et de l'application souhaitée, il est possible de faire varier le diamètre de l'âme, le pas de vis et la hauteur de fil et la présente demande couvre ces divers modes de réalisation. De plus, dans certains modes de réalisation, certains filets mécaniques, comme par exemple les filets trapézoïdaux, offrent moins de résistance ce qui facilite la pénétration de la tige dans le corps tubulaire ancré dans l'os. En effet, un filet trapézoïdal permet de répartir une charge importante en compression et en traction, ce qui améliore la stabilité de l'implant dans le temps et quelles que soient les conditions. Dans certains modes de réalisation, le corps tubulaire (2) comprend sur sa portion distale (23) une portion tronconique (291) dont le diamètre intérieur est inférieur au diamètre extérieur de la tige (1).

Dans certains modes de réalisation, la portion tronconique (291) présente un filetage (232) avec une âme conique permettant au corps tubulaire (2) de s'enfoncer profondément dans l'os.

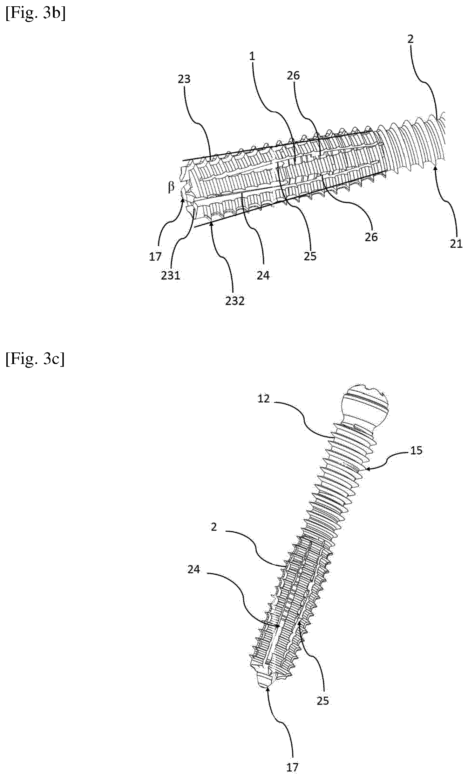

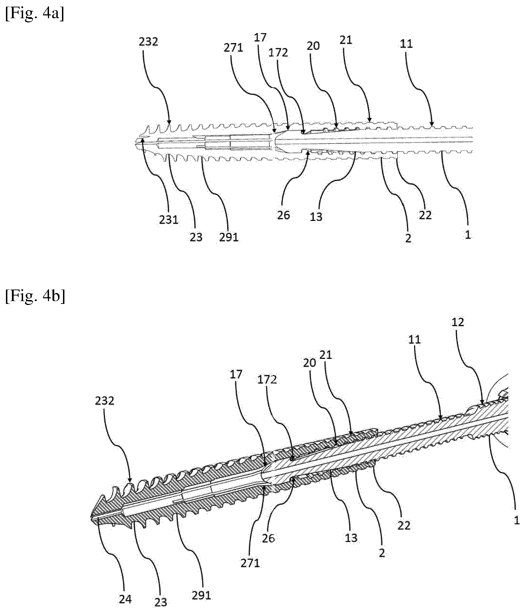

Dans certains modes de réalisation, la portion distale (23) du corps tubulaire (2) est auto-taraudante et comporte des encoches (231) auto-taraudantes (fraisantes et taraudantes), comme par exemple représenté sur [Fig. 3a], [Fig. 3b], [Fig. 4a] et [Fig. 4b]. Cette portion distale (23) permet de préserver l'os lors de l'implantation tout en évitant de pré-percer avant l'insertion de l'implant, et ainsi de conserver une quantité maximale d'os autour de la zone implantée, ce qui améliore la stabilité de l'implant osseux. En effet, le délai d'ostéo-intégration est ainsi réduit ce qui limite la nécessité de rajouter tout type de matériau de comblement osseux, qu'il soit synthétique ou naturel. De plus, la répartition des encoches (231) assure un bon équilibre sur chacune des parties de la portion distale (23) et ainsi une bonne homogénéité de la répartition de l'effort lors de l'insertion de l'implant dans le tissu osseux.

Dans certains modes de réalisation, la tige (1) comprend au moins un marqueur de distance (16) pour visualiser le moment où le vissage de la tige (1) dans le corps tubulaire (2) doit être réalisé dans le sens opposé au vissage du corps tubulaire (2) dans le tissu osseux.

Dans certains modes de réalisation, le marqueur de distance (16) est un marquage laser servant de repère de positionnement pour le praticien lors de la pénétration de l'implant osseux dans l'os, comme cela est représenté par exemple sur [Fig. 2].

Il est à noter que l'implant osseux est réalisé en titane ou en acier inoxydable médical implantable ou en polyéthercétonecétone (PEKK) ou en polyétheréthercétone (PEEK) ou en tout autre matériau dont l'homme du métier pourra déterminer l'adéquation en fonction de ses propriétés mécaniques. physico-chimiques et de sa biocompatibilité.

La présente demande concerne également un procédé d'implantation d'un implant tel que décrit dans les divers modes de réalisation de la présente demande.

Dans certains modes de réalisation, un tel procédé comprend les étapes suivantes : Le vissage de l'implant osseux dans le sens du filetage extérieur (21) jusqu'à ce que le marqueur de distance (16) affleure la surface de la corticale osseuse,

Le vissage de l'implant osseux en vissant dans le sens du second filetage (11) pour compléter le vissage du corps de la tige filetée (1) dans l'os et procéder à l'expansion du corps tubulaire (2).

Dans certains modes de réalisation du procédé, le procédé comprend le ménagement d'une ouverture pour permettre l'insertion de l'implant au travers de l'os cortical au moyen d'un outil de préforme corticale. C'est le cas notamment lorsque le matériau utilisé pour réaliser l'implant est du PEEK.

Dans d'autres modes de réalisation, cette perforation n'est pas nécessaire grâce aux encoches (231) auto-taraudantes.

La présente demande concerne également l'expansion d'un implant osseux dans le tissu osseux.

Dans certains modes de réalisation, comme par exemple représenté sur [Fig. 3a], le corps tubulaire (2) présente un angle aigu α à l'extrémité de sa portion distale (23). Cet angle α s'ouvre et augmente au fur et à mesure que la tige (1) pénètre dans le corps tubulaire (2), lors de l'expansion.

Dans certains modes de réalisation, comme par exemple représenté sur [Fig. 3b], l'angle α en s'ouvrant de plus en plus lors de l'expansion devient un angle β, l'angle β étant l'angle du corps tubulaire (2) expandu.

On notera qu'en position déployée, les parois du corps tubulaire (2) peuvent dans certains modes de réalisation être parallèles au lieu de créer un angle β.

Dans certains modes de réalisation, le corps tubulaire (2) présente une forme bombée au niveau du point d'appui central, comme par exemple représenté sur [Fig. 3c], par la présence des angles α et β. Dans ces modes de réalisation, l'implant présente donc, en configuration expandue, un diamètre externe au niveau du point d'appui central qui est supérieur aux diamètres externes de l'implant au niveau des portions distale et proximale, comme détaillé ci-après.

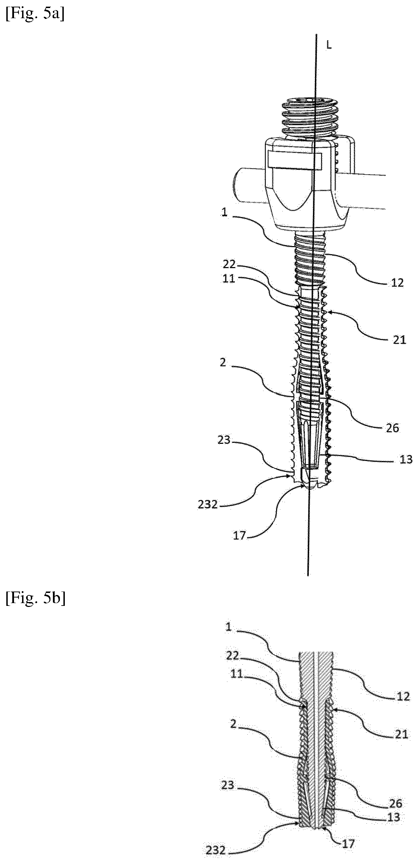

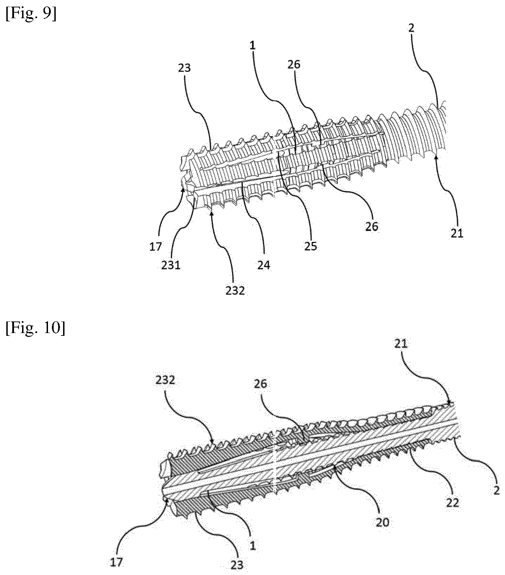

Dans certains modes de réalisation, comme par exemple représenté sur [Fig. 5a], [Fig. 5b] et [Fig. 10], l'implant est expansible entre, d'une part, une configuration de repos dans laquelle un mécanisme de butée verrouille réciproquement le corps tubulaire (2) et ladite tige (1) grâce à l'inversion de leurs deux pas de vis respectifs, et, d'autre part, une configuration expandue obtenue par l'actionnement desdits filetages complémentaires intérieur et extérieur du corps tubulaire (2) et de la tige (1) réciproquement, provoquant la pénétration de la tige (1) dans le corps tubulaire (2) et engendrant l'expansion dudit corps tubulaire (2), grâce au diamètre extérieur de la tige (1) qui est supérieur au diamètre intérieur du corps tubulaire (2), au moins sur une portion distale, par déformation du corps tubulaire (2) lors de la pénétration de la tige (1) dans le corps tubulaire (2).

Comme par exemple représenté sur [Fig. 5a] à [Fig. 10], les profils extérieur de la tige (1) et intérieur du corps tubulaire (2) sont complémentaires, de sorte que dans certains modes de réalisation ils fournissent, en configuration expandue :
Un point d'appui proximal supporté par la complémentarité du diamètre externe de la tige (1) avec le diamètre interne du corps tubulaire (2), ce point d'appui proximal n'entraîne pas la déformation du corps tubulaire (2),
Un point d'appui distal supporté pas la coopération entre le corps tubulaire (2) dont le diamètre interne se rétrécit vers la portion distale jusqu'à être inférieur au diamètre externe de la tige (1),
Un point d'appui « central » localisé entre les deux points d'appui, distal et proximal, formé par la coopération entre le diamètre externe de la tige (1) et le diamètre interne du corps tubulaire (2) qui induisent, en configuration expandue, un diamètre externe du corps tubulaire (2) au niveau « central » qui est supérieur au diamètre externe du corps tubulaire (2) au niveau du point d'appui proximal, ledit point d'appui central n'étant pas nécessairement situé exactement au milieu des deux autres points d'appui.

Dans certains modes de réalisation, le point d'appui distal est formé par la portion distale (13) de la tige comprenant une pointe (17), dont le profil extérieur est complémentaire du profil intérieur de la portion distale (23) du corps tubulaire (2).

Dans certains modes de réalisation, la portion distale (13) est arrêtée en translation dans le sens du filetage extérieur (21) par la butée réciproque configurée dans le corps tubulaire (2), la forme de ladite butée réciproque et la forme intérieure du corps tubulaire (2) étant configurées pour provoquer l'expansion radiale du corps tubulaire (2) lorsque la tige (1) est vissée dans le corps tubulaire (2).

Dans certains modes de réalisation, l'expansion de la portion distale de l'implant est assurée par la coopération entre le profil conique ou tronconique de la pointe (17) de la tige (1) et le diamètre intérieur du corps tubulaire (2), tandis que l'expansion du point d'appui central est assurée par la coopération entre le diamètre intérieur au niveau du point d'appui central du corps tubulaire (2) par rapport à l'augmentation du diamètre extérieur de la tige (1), à partir de la butée réciproque en direction de la portion proximale.

De préférence, cette augmentation du diamètre extérieur de la tige (1) est située à une distance prédéterminée par rapport au marqueur de distance (16), ainsi il est possible de visser l'implant en position de repos jusqu'audit marqueur de distance (16) puis de visser en sens inverse pour permettre la pénétration de la tige à l'intérieur du manchon suivie de l'expansion du corps tubulaire (2), tout en contrôlant la profondeur à laquelle l'expansion aura lieu.

De plus, le diamètre extérieur de la tige (1) est supérieur au diamètre intérieur du corps tubulaire (2), par l'intermédiaire d'au moins un rétrécissement (271) sur une portion distale (23), le diamètre extérieur de la tige (1) atteignant sa taille maximale à la distance le long de l'axe longitudinal (L) correspondant au point d'appui central en position expandue. Ledit au moins un rétrécissement (271) est situé, par rapport à la portion proximale et selon l'axe longitudinal (L), à une distance déterminée en fonction de la profondeur, dans le tissu osseux, à laquelle ladite expansion est souhaitée, comme cela est représenté par exemple sur [Fig. 4a] et [Fig. 4b].

Ledit rétrécissement (271) est situé à une distance variable de la pointe (17).

Dans certains modes de réalisation, le mécanisme de butée réciproque comporte une saillie (26) (ou nervure faisant saillie), appelée nervure de façon non limitative, à l'intérieur du corps tubulaire (2), complémentaire d'un épaulement ou d'une entaille (172) situé sur la pointe de la tige (1).

Dans certains modes de réalisation, le diamètre de la tige (1) augmente progressivement depuis ledit épaulement ou ladite entaille (172), vers la portion distale (13) de la tige (1) pour permettre au point d'appui central d'expandre le corps tubulaire (2).

Dans certains modes de réalisation, le point d'appui proximal est formé sur au moins une portion de la portion distale (23) complémentaire du profil extérieur de la tige (1).

Ce point d'appui proximal correspond à une complémentarité de diamètres entre le corps tubulaire (2) et la tige (1).

Dans certains modes de réalisation, le point d'appui « central » est formé par au moins une saille (26) (par exemple une nervure faisant saillie) à l'intérieur du corps tubulaire (2), sur une portion intermédiaire entre la portion distale (23) et la portion proximale (22), de sorte que ladite nervure (26) coopère, lors de l'expansion, avec la surface extérieure de la tige (1) entre sa portion proximale et sa portion distale. Dans certains modes de réalisation, le diamètre externe du corps tubulaire (2) au niveau « central » est aussi, de préférence, supérieur au diamètre externe du corps tubulaire (2) au niveau du point d'appui distal, ce qui induit la forme bombée décrite ci-dessus (du corps tubulaire (2) et de l'implant en général) au niveau du point d'appui central, par exemple comme représenté sur [Fig. 3c] et [Fig. 10]. Dans ces modes de réalisation, la forme bombée en configuration expandue est obtenue par le fait que la coopération entre les extrémités distales de la tige (1) et du corps tubulaire (2) impose un diamètre extérieur à l'implant qui est inférieur à celui imposé au niveau de l'appui central détaillé dans la présente demande. De plus, cette expansion selon une forme bombée est facilitée dans certains modes de réalisation par la présence de fentes non débouchantes (25) sur une portion centrale du corps tubulaire, comme détaillé dans la présente demande (ces fentes non débouchantes (25) présentant de plus l'avantage de permettre une croissance de tissu osseux dans l'implant pour améliorer la stabilité de l'implant).

De plus, dans certains modes de réalisation, un effet de synergie est observé entre la butée réciproque et le point d'appui « central » permettant le vissage au départ mais aussi l'appui lors de l'expansion.

Dans certains modes de réalisation, le point d'appui central et le mécanisme de butée verrouillant réciproquement le corps tubulaire (2) et la tige (1) sont formés par les mêmes éléments (ou du moins des éléments communs pour les deux mécanismes) coopérant, respectivement, en configuration expandue ou en configuration de repos. Ces mêmes éléments coopérants forment ensemble une même structure réalisant deux fonctions différentes selon la configuration. En effet, ces éléments qui comprennent la nervure (26) et le profil extérieur de la tige (avec l'entaille (172) à proximité de son extrémité distale) coopèrent, d'une part, en configuration de repos, de façon à former la butée réciproque (permettant le vissage dans l'os) et, d'autre part, en configuration expandue, de manière à réaliser le point d'appui central (permettant l'expansion de l'implant). En formant une butée contre l'entaille (172), la nervure (26) permet de retenir la tige (1) par rapport au corps tubulaire (2) pendant le vissage de l'implant dans l'os et lors du vissage en sens inverse (de la tige (1) dans le corps tubulaire (2)) grâce au pas de vis inversé du filetage (11) extérieur de la tige (1) par rapport au second filetage extérieur (21) du corps tubulaire (2) comme détaillé ci-dessus, la coopération de la nervure (26) avec le profil extérieur de la tige (1) (qui augmente en direction de l'extrémité proximale) induit l'expansion du corps tubulaire (2). On comprend donc que ces deux effets techniques de vissage et d'expansion centrale sont obtenus par des éléments communs présentant une synergie, ce qui fournit divers avantages pour l'implant (simplicité et coût de fabrication, stabilité, etc.)

Dans certains modes de réalisation, le mécanisme de butée réciproque comporte en outre une nervure ou un épaulement ou une saillie à l'intérieur du corps tubulaire (2), complémentaire du diamètre extérieur de la tige (1), ou une nervure sur l'intérieur du corps tubulaire (2), complémentaire d'une rainure ou d'un épaulement sur la tige (1). Les points d'appui au niveau de l'effort d'expansion maximale limitent les risques d'affaissement sur les parties cumulant le plus d'efforts de contacts entre l'os et l'implant.

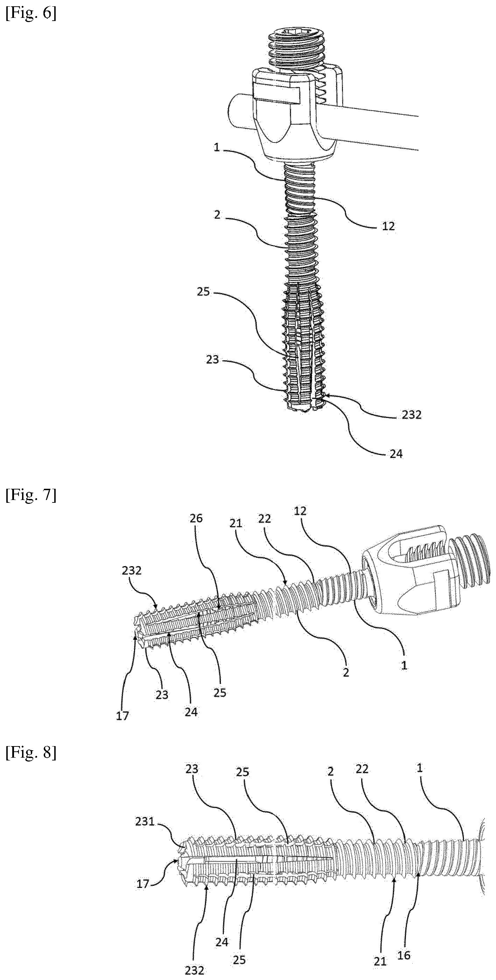

Comme par exemple représenté sur [Fig. 3a] à [Fig. 3c], et [Fig. 6] à [Fig. 10] la portion distale (23) du corps tubulaire (2) comporte notamment des fentes longitudinales débouchantes (24) et non débouchantes (25) pour permettre l'expansion cylindrique du corps tubulaire (2).

Dans certains modes de réalisation, la synergie entre les fentes débouchantes (24) et les fentes non débouchantes (25) permet en outre une géométrie de cône tronqué. Dans certains modes de réalisation, l'expansion est permise grâce à au moins une fente longitudinale débouchante (24) ou non débouchante (25), de préférence plusieurs fentes débouchantes (24) ou non débouchantes (25). Il est également préférable que la portion distale (23) comporte les deux types de fentes, c'est-à-dire des fentes longitudinales débouchantes (24) et des fentes longitudinales non débouchantes (25). Il est à noter que la répartition des encoches (231) de la portion (23) auto-taraudante du corps tubulaire (2) dépend du nombre de fentes longitudinales débouchantes (24) et non débouchantes (25). Les fentes longitudinales non débouchantes (25) et le point d'appui central autorisent l'expansion du corps tubulaire (2).

Dans certains modes de réalisation, il y a autant d'encoches (231) auto-taraudantes que de fentes longitudinales débouchantes (24).

Comme par exemple représenté sur [Fig. 5a] à [Fig. 10] le corps tubulaire (2) est expandu après pénétration de la tige (1) à l'intérieur du corps tubulaire (2).

Dans certains modes de réalisation, le corps tubulaire (2) s'expand sous forme cylindrique et les fentes longitudinales débouchantes (24) et non débouchantes (25) sur la portion distale (23) du corps tubulaire (2) permettent l'expansion cylindrique du corps tubulaire (2) en permettant une déformation élastique ou plastique du corps tubulaire (2) lors de la pénétration de la tige (1) dans le corps tubulaire (2). Le corps tubulaire (2) peut être réalisé par exemple en titane qui présente de bonnes propriétés de déformation plastique. Les fentes longitudinales non débouchantes (25) contribuent ainsi à la stabilité de l'implant osseux dans le tissu osseux en permettant lors de l'expansion de pouvoir conserver le profil de contact sur les trois points d'appui entre le corps tubulaire (2) et la tige (1), et en laissant les efforts dus à l'expansion se répartir de façon homogène sur la périphérie du corps tubulaire (2) expandu.

Dans certains modes de réalisation, les fentes débouchantes (24) et les fentes non débouchantes (25) sont disposées de façon décalée les unes par rapport aux autres sur la longueur, cette disposition décalée permet d'améliorer la souplesse et la résistance mécanique du corps tubulaire (2) lors de l'expansion.

Enfin, dans certains modes de réalisation, les fentes non débouchantes (25) permettent au corps tubulaire (2) de s'expandre dans le tissu osseux spongieux en présentant une forme bombée, de type convexe, permettant de comprimer et densifier la matière sur sa périphérie, améliorant ainsi la stabilité primaire, la cicatrisation, et permettant d'éviter l'ajout de ciment pour stabiliser et immobiliser l'implant osseux.

L'implant osseux proposé dans l'invention peut donc être implanter rapidement et de façon précise dans le tissu osseux, et rester implanter de façon très stable dans le tissu osseux.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un ou plusieurs autre(s) mode(s) de réalisation à moins que l'inverse ne soit explicitement mentionné ou que ces caractéristiques ne soient incompatibles ou que la combinaison ne fonctionne pas. Plus généralement, des combinaisons de divers types de moyen de retenue de l'implant et/ou de moyen de retenue des épineuses sont envisagées et seront appréciées par l'homme de métier à l'aide des considérations fonctionnelles et structurelles fournies dans la présente demande. De plus, les caractéristiques techniques décrites dans un mode de réalisation donné peuvent être isolées des autres caractéristiques de ce mode à moins que l'inverse ne soit explicitement mentionné, notamment car les considérations fonctionnelles fournies dans la présente demande fourniront une explication suffisante pour que les adaptations structurelles éventuellement nécessaires soient à la portée de l'homme de métier.

Les personnes versées dans l'art, à la lecture de la présente demande, comprendront que des modes de réalisation sous de nombreuses autres formes spécifiques que celles décrites en détail sont possibles sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit être limitée aux détails donnés ci-dessus.

## Revendications

1. Implant d'ancrage osseux à expansion optimisée, comprenant :
Un corps tubulaire (2) s'étendant entre une portion proximale (22) possédant un premier diamètre intérieur, et une portion distale (23) possédant un second diamètre intérieur inférieur audit premier diamètre intérieur, ces deux portions définissant un axe longitudinal (L) et lesdits premier et second diamètres intérieur définissant un profil intérieur dudit corps tubulaire (2), et comprenant,
d'une part, au moins un premier filetage (20) à l'intérieur du corps tubulaire (2) et, d'autre part, au moins un second filetage (21) à l'extérieur du corps tubulaire (2),
Une tige (1) s'étendant entre une portion proximale (12) et une portion distale (13) sur un axe colinéaire à l'axe (L) et présentant, d'une part, le long dudit axe longitudinal (L), un profil extérieur complémentaire audit profil intérieur dudit corps tubulaire (2) et, d'autre part, au moins un filetage (11) extérieur dont le pas de vis est inversé par rapport audit second filetage extérieur (21) du corps tubulaire (2),
L'implant étant expansible entre, d'une part, une configuration de repos dans laquelle un mécanisme de butée verrouille réciproquement ledit corps tubulaire (2) et ladite tige (1) grâce à l'inversion de ces deux pas de vis, et,
d'autre part, une configuration expandue obtenue par l'actionnement desdits filetages complémentaires intérieur et extérieur du corps tubulaire (2) et de la tige (1) réciproquement, provoquant la pénétration de la tige (1) dans le corps tubulaire (2) et engendrant l'expansion dudit corps tubulaire (2), grâce au diamètre extérieur de la tige (1) qui est supérieur au diamètre intérieur du corps tubulaire (2), au moins sur une portion distale, par déformation du corps tubulaire (2) lors de la pénétration de la tige (1) dans le corps tubulaire (2),
Les profils extérieur de la tige (1) et intérieur du corps tubulaire (2) sont complémentaires, de sorte qu'ils fournissent, en configuration expandue :
Un point d'appui proximal supporté par la complémentarité du diamètre externe de la tige (1) avec le diamètre interne du corps tubulaire (2),
Un point d'appui distal supporté pas la coopération entre le corps tubulaire (2) dont le diamètre interne se rétrécit vers la portion distale jusqu'à être inférieur au diamètre externe de la tige (1),
Un point d'appui « central » localisé entre ces deux points d'appuis (au niveau proximal et distal), formé par la coopération entre le diamètre externe de la tige (1) et le diamètre interne du corps tubulaire (2) qui induisent, en configuration expandue, un diamètre externe du corps tubulaire (2) au niveau « central » qui est supérieur au diamètre externe du corps tubulaire (2) au niveau du point d'appui proximal.

2. Implant selon la revendication 1, **caractérisé en ce que** ledit diamètre externe du corps tubulaire (2) au niveau « central » est supérieur au diamètre externe du corps tubulaire (2) au niveau du point d'appui distal.

3. Implant selon la revendication 1, **caractérisé en ce que** le point d'appui distal est formé sur au moins une portion de la portion distale (23) complémentaire du profil extérieur de la tige (1).

4. Implant selon la revendication 2, **caractérisé en ce que** le point d'appui distal est formé par ladite portion distale (13) comprenant une pointe (17) dont le profil extérieur est complémentaire du profil intérieur de la portion distale (23) du corps tubulaire (2).

5. Implant selon l'une des revendications 1 à 4, **caractérisé en ce que** le point d'appui central est formé par au moins une nervure (26) faisant saillie à l'intérieur du corps tubulaire (2), sur une portion intermédiaire entre la portion distale (23) et la portion proximale (22), de sorte que ladite nervure (26) coopère, lors de l'expansion, avec la surface extérieure de la tige (1), entre sa portion proximale (12) et sa portion distale (13).

6. Implant selon l'une des revendications 1 à 5, **caractérisé en ce que** le mécanisme de butée réciproque comporte une nervure ou un épaulement ou une saillie à l'intérieur du corps tubulaire (2), complémentaire du diamètre extérieur de la tige (1), ou une nervure sur l'intérieur du corps tubulaire (2), complémentaire d'une rainure ou d'un épaulement sur la tige (1).

7. Implant selon la revendication 6, **caractérisé en ce que** le mécanisme de butée réciproque comporte une nervure (26) faisant saillie à l'intérieur du corps tubulaire (2) complémentaire d'un épaulement ou d'une entaille (172) situé sur la pointe de la tige (1).

8. Implant selon la revendication 7, **caractérisé en ce que** le point d'appui central et le mécanisme de butée verrouillant réciproquement le corps tubulaire (2) et la tige (1) sont formés par les mêmes éléments coopérant, respectivement, en configuration expandue ou en configuration de repos.

9. Implant selon les revendications précédentes prises dans leur ensemble, **caractérisé en ce que** le diamètre extérieur de la tige (1) est supérieur au diamètre intérieur du corps tubulaire (2). par au moins un rétrécissement (271) sur une portion distale.

10. Implant selon la revendication 9, **caractérisé en ce que** ledit au moins un rétrécissement (271) est situé, par rapport à la portion proximale et selon l'axe longitudinal (L), à une distance déterminée en fonction de la profondeur, dans le tissu osseux, à laquelle ladite expansion est souhaitée.

11. Implant selon l'une des revendications 1 à 9, **caractérisé en ce que** le corps tubulaire (2) comprend sur sa portion distale (23) une portion tronconique (291) dont le diamètre intérieur est inférieur au diamètre extérieur de la tige (1).

12. Implant selon la revendication 11, **caractérisé en ce que** la portion tronconique (291) présente un filetage (232) avec une âme conique permettant au corps tubulaire (2) de s'enfoncer profondément dans l'os.

13. Implant selon les revendications précédentes prises dans leur ensemble, **caractérisé en ce que** la portion distale (23) comporte des encoches (231) auto-taraudantes.

14. Implant selon la revendication 11, **caractérisé en ce que** la portion distale (23) comporte des fentes longitudinales débouchantes (24) permettant l'expansion du corps tubulaire (2).

15. Implant selon la revendication 13, **caractérisé en ce qu'**il y a autant d'encoches (231) auto-taraudantes que de fentes longitudinales débouchantes (24).

16. Implant selon la revendication 11, **caractérisé en ce que** la portion distale (23) comporte des fentes longitudinales non débouchantes (25) permettant l'expansion du corps tubulaire (2).

17. Implant selon l'une quelconque des revendications précédentes, **caractérisé en ce que** ladite tige (1) comprend au moins un marqueur de distance (16) pour visualiser le moment où le vissage de la tige (1) dans le corps tubulaire (2) doit être réalisé dans le sens opposé au vissage du corps tubulaire (2) dans le tissu osseux.

## Patentansprüche

1. Knochenverankerungsimplantat mit optimierter Expansion, umfassend:
einen röhrenförmigen Körper (2), der sich zwischen einem proximalen Abschnitt (22) mit einem ersten Innendurchmesser und einem distalen Abschnitt (23) mit einem zweiten Innendurchmesser, der kleiner als der erste Innendurchmesser ist, erstreckt, wobei diese beiden Abschnitte eine Längsachse (L) definieren und der erste und zweite Innendurchmesser ein Innenprofil des röhrenförmigen Körpers (2) definieren, und einerseits mindestens ein erstes Gewinde (20) an der Innenseite des röhrenförmigen Körpers (2) und andererseits mindestens ein zweites Gewinde (21) an der Außenseite des röhrenförmigen Körpers (2) umfasst,
eine Schaft (1), der sich zwischen einem proximalen Abschnitt (12) und einem distalen Abschnitt (13) auf einer zu der Achse (L) kollinearen Achse erstreckt und einerseits entlang der Längsachse (L) ein externes Profil, das zu dem inneren Profil des röhrenförmigen Körpers (2) komplementär ist, und andererseits mindestens ein Außengewinde (11) aufweist, dessen Gewindesteigung gegenüber dem zweiten Außengewinde (21) des röhrenförmigen Körpers (2) umgekehrt ist,
wobei das Implantat zwischen einerseits einer Ruhekonfiguration, in der ein Anschlagmechanismus den röhrenförmigen Körper (2) und den Schaft (1) durch die Umkehrung dieser beiden Gewindesteigungen gegenseitig verriegelt, und andererseits einer expandierten Konfiguration expandierbar ist, die durch die gegenseitige Betätigung des komplementären Innen- und
Außengewindes des röhrenförmigen Körpers (2) und des Schafts (1) erhalten wird, wodurch der Schaft (1) in den röhrenförmigen Körper (2) eindringt und die Expansion des röhrenförmigen Körpers (2) dank des Außendurchmessers des Schafts (1), der mindestens an einem distalen Abschnitt größer als der Innendurchmesser des röhrenförmigen Körpers (2) ist, durch Verformung des röhrenförmigen Körpers (2) erzeugt wird, wenn der Schaft (1) in den röhrenförmigen Körper (2) eindringt,
wobei die äußeren Profile des Schafts (1) und die inneren Profile des röhrenförmigen Körpers (2) komplementär sind,
dass sie in der expandierten Konfiguration Folgendes bereitstellen:
einen proximalen Auflagepunkt, der durch die Komplementarität des Außendurchmessers des Schafts (1) zu dem Innendurchmesser des röhrenförmigen Körpers (2) gestützt wird,
einen distalen Auflagepunkt, der durch das Zusammenwirken zwischen dem röhrenförmigen Körper (2) gestützt wird, dessen Innendurchmesser sich zu dem distalen Abschnitt hin verengt, bis er kleiner als der Außendurchmesser des Schafts (1) ist,
einen "mittleren" Auflagepunkt zwischen diesen beiden (proximalen und distalen) Auflagepunkten, der durch das Zusammenwirken zwischen dem Außendurchmesser des Schafts (1) und dem Innendurchmesser des röhrenförmigen Körpers (2) gebildet wird, die in der expandierten Konfiguration einen Außendurchmesser des röhrenförmigen Körpers (2) auf "mittlerer" Höhe bewirken, der auf der Höhe des proximalen Auflagepunkts größer als der Außendurchmesser des röhrenförmigen Körpers (2) ist.

2. Implantat nach Anspruch 1, **dadurch gekennzeichnet, dass** der Außendurchmesser des röhrenförmigen Körpers (2) auf der "mittleren" Höhe größer als der Außendurchmesser des röhrenförmigen Körpers (2) auf der Höhe des distalen Auflagepunkts ist.

3. Implantat nach Anspruch 1, **dadurch gekennzeichnet, dass** der distale Auflagepunkt an mindestens einem Teil des distalen Abschnitts (23) ausgebildet ist, der zu dem äußeren Profil des Schafts (1) komplementär ist.

4. Implantat nach Anspruch 2, **dadurch gekennzeichnet, dass** der distale Auflagepunkt durch den distalen Abschnitt (13) gebildet wird, der eine Spitze (17) aufweist, deren Außenprofil zu dem Innenprofil des distalen Abschnitts (23) des röhrenförmigen Körpers (2) ist.

5. Implantat nach einem der Ansprüche 1 bis 4, **dadurch gekennzeichnet, dass** der mittlere Auflagepunkt durch mindestens eine Rippe (26) gebildet ist, die in das Innere des röhrenförmigen Körpers (2) ragt, auf einem Zwischenabschnitt zwischen dem distalen Abschnitt (23) und dem proximalen Abschnitt (22), so dass die Rippe (26) während der Expansion mit der Außenfläche des Schafts (1) zwischen seinem proximalen Abschnitt (12) und seinem distalen Abschnitt (13) zusammenwirkt.

6. Implantat nach einem der Ansprüche 1 bis 5, **dadurch gekennzeichnet, dass** der gegenseitige Anschlagmechanismus eine Rippe oder eine Schulter oder einen Vorsprung in das Innere des röhrenförmigen Körpers (2) aufweist, die bzw. der zu dem Außendurchmesser des Schafts (1) komplementär ist, oder eine Rippe an der Innenseite des röhrenförmigen Körpers (2), die zu einer Nut oder einer Schulter an dem Schaft (1) komplementär ist.

7. Implantat nach Anspruch 6, **dadurch gekennzeichnet, dass** der gegenseitige Anschlagmechanismus eine Rippe (26) umfasst, die in das Innere des röhrenförmigen Körpers (2) ragt und zu einer Schulter oder einer Einkerbung (172) an der Spitze des Schafts (1) komplementär ist.

8. Implantat nach Anspruch 7, **dadurch gekennzeichnet, dass** der mittlere Auflagepunkt und der Anschlagmechanismus, der den röhrenförmigen Körper (2) und den Schaft (1) gegenseitig verriegelt, durch dieselben Elemente gebildet sind, die jeweils in der expandierten Konfiguration bzw. in der Ruhekonfiguration zusammenwirken.

9. Implantat nach den vorhergehenden Ansprüchen als Ganzes genommen, **dadurch gekennzeichnet, dass** der Außendurchmesser des Schafts (1) durch mindestens eine Verengung (271) an einem distalen Abschnitt größer als der Innendurchmesser des röhrenförmigen Körpers (2) ist.

10. Implantat nach Anspruch 9, **dadurch gekennzeichnet, dass** die mindestens eine Verengung (271) in Bezug auf den proximalen Abschnitt und entlang der Längsachse (L) mit einem von der Tiefe bestimmten Abstand im Knochengewebe angeordnet ist, bei der die Expandierung gewünscht ist.

11. Implantat nach einem der Ansprüche 1 bis 9, **dadurch gekennzeichnet, dass** der röhrenförmige Körper (2) an seinem distalen Abschnitt (23) einen kegelstumpfförmigen Abschnitt (291) umfasst, dessen Innendurchmesser kleiner als der Außendurchmesser des Schafts (1) ist.

12. Implantat nach Anspruch 11, **dadurch gekennzeichnet, dass** der kegelstumpfförmige Abschnitt (291) ein Gewinde (232) mit einem konischen Kern aufweist, wodurch der röhrenförmige Körper (2) tief in den Knochen sinken kann.

13. Implantat nach den vorhergehenden Ansprüchen als Ganzes genommen, **dadurch gekennzeichnet, dass** der distale Abschnitt (23) selbstschneidende Kerben (231) aufweist.

14. Implantat nach Anspruch 11, **dadurch gekennzeichnet, dass** der distale Abschnitt (23) durchgehende Längsschlitze (24) aufweist, die die Expandierung des röhrenförmigen Körpers (2) gestatten.

15. Implantat nach Anspruch 13, **dadurch gekennzeichnet, dass** so viele selbstschneidende Kerben (231) wie durchgehende Längsschlitze (24) vorliegen.

16. Implantat nach Anspruch 11, **dadurch gekennzeichnet, dass** der distale Abschnitt (23) nicht durchgehende Längsschlitze (25) aufweist, die die Expandierung des röhrenförmigen Körpers (2) gestatten.

17. Implantat nach einem der vorhergehenden Ansprüche, **dadurch gekennzeichnet, dass** der Schaft (1) mindestens eine Abstandsmessmarke (16) umfasst, um den Zeitpunkt darzustellen, an dem das Einschrauben des Schafts (1) in den röhrenförmigen Körper (2) in entgegengesetzter Richtung zum Einschrauben des röhrenförmigen Körpers (2) in das Knochengewebe erfolgen muss.

## Claims

1. An osseous anchoring implant with optimized expansion, comprising:
A tubular body (2) extending between a proximal portion (22) having a first internal diameter, and a distal portion (23) having a second internal diameter smaller than said first internal diameter, these two portions defining a longitudinal axis (L) and said first and second internal diameters defining an internal profile of said tubular body (2), and comprising, on the one hand, at least a first threading (20) inside the tubular body (2) and, on the other hand, at least a second threading (21) outside the tubular body (2),
A rod (1) extending between a proximal portion (12) and a distal portion (13) on an axis collinear with the axis (L) and having, on the one hand, along said longitudinal axis (L), an external profile complementary to said internal profile of said tubular body (2) and, on the other hand, at least one external threading (11) whose screw pitch is reversed relative to said second external threading (21) of the tubular body (2),
The implant being expandable between, on the one hand, a rest configuration in which an abutment mechanism interlocks said tubular body (2) and said rod (1) thanks to the reversal of these two screw pitches and, on the other hand, an expanded configuration obtained by the actuation of said complementary internal and external threadings of the tubular body (2) and of the rod (1) mutually, causing the penetration of the rod (1) into the tubular body (2) and generating the expansion of said tubular body (2), thanks to the external diameter of the rod (1) which is greater than the internal diameter of the tubular body (2), at least on a distal portion, by deformation of the tubular body (2) during penetration of the rod (1) into the tubular body (2),
The external profile of the rod (1) and the internal profile of the tubular body (2) are complementary, so that they provide, in an expanded configuration:
A proximal bearing supported by the complementarity of the outer diameter of the rod (1) with the inner diameter of the tubular body (2),
A distal bearing supported by the cooperation between the tubular body (2) whose inner diameter narrows towards the distal portion until becoming smaller than the outer diameter of the rod (1),
A "central" bearing located between these two (proximal and distal) bearings, formed by the cooperation between the outer diameter of the rod (1) and the inner diameter of the tubular body (2) which induce, in the expanded configuration, an outer diameter of the tubular body (2) at the "central" level which is greater than the outer diameter of the tubular body (2) at the proximal bearing.

2. The implant according to claim 1, **characterized in that** the outer diameter of the tubular body (2) at the "central" level is greater than the outer diameter of the tubular body (2) at the distal bearing.

3. The implant according to claim 1, **characterized in that** the distal bearing is formed on at least one portion of the distal portion (23) complementary to the external profile of the rod (1).

4. The implant according to claim 2, **characterized in that** the distal bearing is formed by said distal portion (13) comprising a tip (17) whose external profile is complementary to the internal profile of the distal portion (23) of the tubular body (2).

5. The implant according to any one of claims 1 to 4, **characterized in that** the central bearing is formed by at least one rib (26) protruding inside the tubular body (2), on an intermediate portion between the distal portion (23) and the proximal portion (22) such that said rib (26) cooperates, during the expansion of the implant, with the outer surface of the rod (1) between its proximal portion (12) and its distal portion (13).

6. The implant according to any one of claims 1 to 5, **characterized in that** the mutual abutment mechanism includes a rib or a shoulder or a protrusion inside the tubular body (2), complementary to the external diameter of the rod (1), or a rib on the inside of the tubular body (2), complementary to a groove or to a shoulder on the rod (1).

7. The implant according to claim 6, **characterized in that** the the mutual abutment mechanism includes a rib (26) protruding inside the tubular body (2) complementary to a shoulder or to a cut (160) located on the tip of the rod (1).

8. The implant according to claim 7, **characterized in that** the central bearing and the abutment mechanism interlocking the tubular body (2) and the rod (1) are formed by the same elements cooperating, respectively either in the expanded configuration or the rest configuration.

9. The implant according to the preceding claims combined, **characterized in that** the external diameter of the rod (1) is greater than the internal diameter of the tubular body (2), by at least one shrinkage (271) on a distal portion.

10. The implant according to claim 9, **characterized in that** said at least one shrinkage (271) is located, relative to the proximal portion and along the longitudinal axis (L), at a distance determined as a function of the depth, in the osseous tissue, at which said expansion is desired.

11. The implant according to any of claims 1 to 9, **characterized in that** the tubular body (2) comprises on its distal portion (23) a frustoconical portion (291) whose internal diameter is smaller than the external diameter of the rod (1).

12. The implant according to claim 11, **characterized in that** the frustoconical portion (291) has a threading (232) with a conical core allowing the tubular body (2) to sink deep into the bone.

13. The implant according to the preceding claims combined, **characterized in that** the distal portion (23) includes self-tapping notches (231).

14. The implant according to claim 11, **characterized in that** the distal portion (23) includes longitudinal through-slots (24) allowing the expansion of the tubular body (2).

15. The implant according to claim 13, **characterized in that** there are as many self-tapping notches (231) as there are longitudinal through-slots (24).

16. The implant according to claim 11, **characterized in that** the distal portion (23) includes longitudinal non-through slots (25) allowing the expansion of the tubular body (2).

17. The implant according to any one of the preceding claims, **characterized in that** said rod (1) comprises at least one distance marker (16) to visualize the moment when the screwing of the rod (1) in the tubular body (2) must be carried out in the opposite direction to the screwing of the tubular body (2) into the osseous tissue.
